# EUROPEAN PATENT APPLICATION

(11) **EP 1 121 909 A2**
(43) Date of publication of application: **08.08.2001**
(21) Application number: 01830063.2
(22) Date of filing: 31.01.2001
(51) Int. Cl.: A61C 17/20, A61C 17/22, B06B 1/02

(54) **Improved toothbrush**

(30) Priority: 07.02.2000 IT RM000053
(71) Applicant: Gai, Giorgio, 00167 Roma (IT); Gai, Simona, 00167 Roma (IT)
(72) Inventor: Gai, Giorgio, 00167 Roma (IT); Gai, Simona, 00167 Roma (IT)
(74) Representative: Iannone, Carlo Luigi

(57) **Abstract**

The invention relates to an improved toothbrush (1) comprising a base element (2), an handle (3) and a replaceable toothbrush (4), provided with bristles (5), and an electronic circuit, said circuit comprising a feeding unit (A), provided in said base element (2), a control unit (C), an oscillator (D) generator and mixer of frequencies depending on the clock, provided within the handle (3), said toothbrush (4) acting as antenna.

## Description

The present invention relates to an improved toothbrush.

More specifically, the invention concerns to a toothbrush realised in such a way to mainly allow the whitening of the teeth enamel, with the consequent reduction of the bacterial plaque and the tartar formation.

As it is well known, during last years an evolution occurred as far as the care of teeth is concerned, particularly with reference to the treatment to be made at home, to remove plaque and tartar.

Therefore, a remarkable development occurred of apparatuses allowing to validly remove plaque and tartar using the toothbrush directly at home.

All the known solutions, that in some way constitute a response to the above mentioned needing, cannot carry out a satisfactory removal both for the nature of plaque and tartar, and in view of the fact that they are present in positions very difficult to be reached.

Therefore, notwithstanding the care that each one can take while making this operation, it is unavoidable to periodically go to the teeth cleaning directly with the dentist.

Thus it seems well evident the needing of having available a toothbrush that is, on one side, a more efficient instrument to remove the plaque and/or tartar, and on the other side, represents during its specific daily use, a fast and real proposal in the prevention field, and a real help in the eventual specialist intervention of dental cleaning.

In this field is included the solution proposed according to the present invention, suggesting an improved toothbrush allowing to remove in a very efficient way the bacterial plaque and/or tartar.

These and other results are obtained according to the present invention suggesting a toothbrush realised in such a way to technically work as an antenna, with determined bands and frequency scale, in such a way to act on bacterial plaque and tartar, breaking their linkages and thus allowing an optimum removal.

It is therefore specific object of the present invention an improved toothbrush comprising a base element, an handle and a replaceable toothbrush, provided with bristles, and an electronic circuit, said circuit comprising a feeding, provided in said base element, a control unit, an oscillator generator mixer of frequencies depending on the clock, provided within the handle, said toothbrush acting as antenna.

Preferably, five frequencies included in the range between 0 and 2500 Hz will be generated and mixed.

Still more preferably, said frequencies are 1972.63 Hz, 246.58 Hz, 30.822 Hz, 3852 Hz, 0.481 Hz, with a division constant equal to 8.

Furthermore, according to the invention, a rechargeable battery can be provided.

Still according to the invention an activation pilot lamp can be provided in said handle.

In a preferred embodiment of the toothbrush according to the invention, said circuit provides four stages, namely:
- a recharging tension detector and inhibitor of the oscillation stage;
- a recharging current limiting device and a controller of the battery B tension;
- a generator dividing the clock signal;
- a frequency generator and mixer, preferably five frequencies, depending on the clock.

The present invention will be now described, for illustrative but not limitative purposes, according to its preferred embodiments, with particular reference to the figures of the enclosed drawings, wherein:
figure 1 is a perspective view of an embodiment of a toothbrush according to the invention, assembled;
figure 2 shows the toothbrush of figure 1 assembled;
figures 3a and 3b show block diagrams for the toothbrush according to the invention;
figure 4 shows an embodiment of a lay out of a circuit for the toothbrush according to the invention; and
figure 5 shows an embodiment of a circuit scheme for the toothbrush according to the invention.

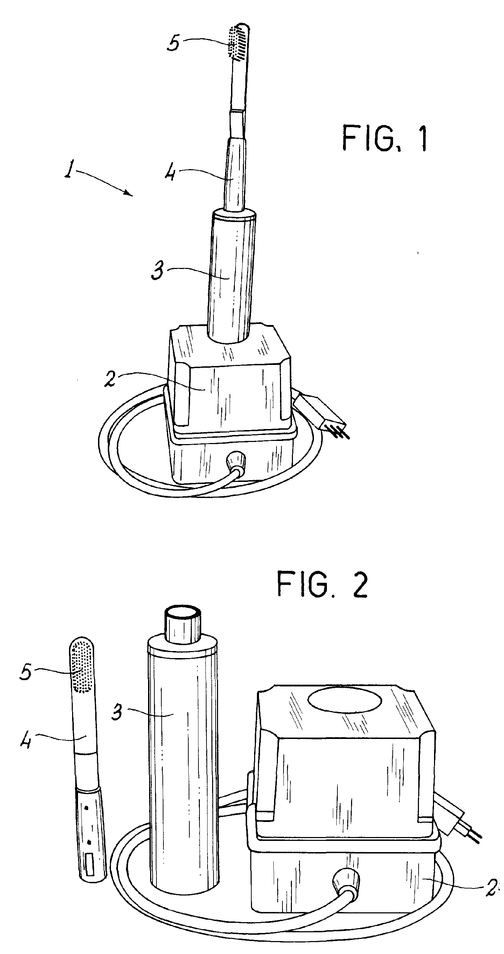

Observing the enclosed figures, and first figures 1 and 2, for illustrative but not limitative purposes, since the specific structure is not a peculiar feature of the invention, a toothbrush 1 is shown, comprising a base 2, a handle 3 and a replaceable toothbrush 4.

Within the base 2 a feeding transformer is provided, comprised only of the primary winding, since the secondary winding is provided within the handle 3. Within said handle 3, beside the secondary winding, for feeding, also the electronic circuitry and the feeding battery are provided.

The toothbrush 4 has an electrical coupling between the handle 3 circuit and the metallic base 4 of the bristles 5.

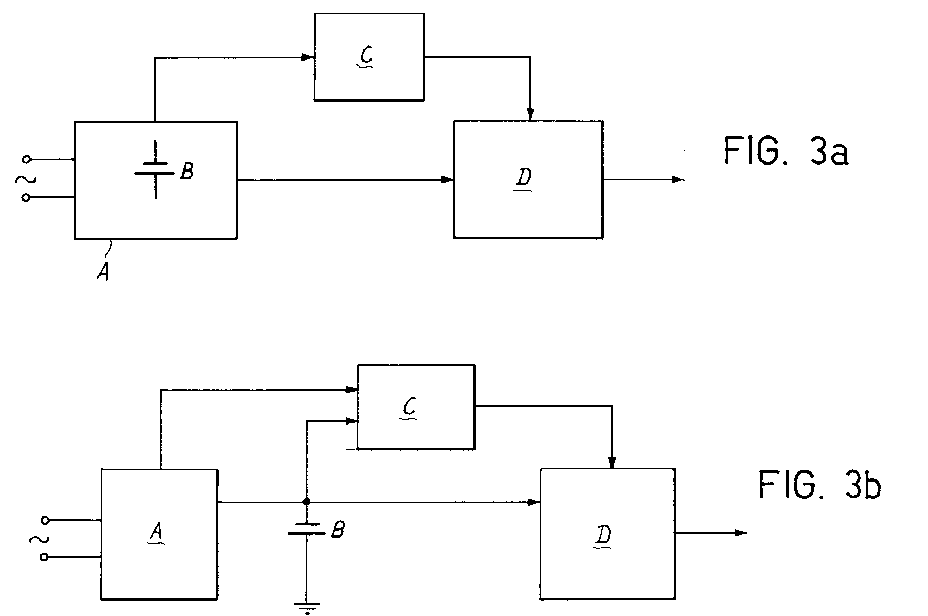

In figures 3a and 3b two possible block diagrams of the circuit for the toothbrush according to the invention are provided.

Particularly, in figure 3a it is provided a feeding A, with a built-in battery B, coupled, on one side, with a control circuit C, and on the other side, with an oscillator D, provided within the toothbrush 4. Said control circuit C provides an enabling - de-enabling system.

In the embodiment of figure 3b, battery B is provided out of the feeding A.

Coming now to describe the operation of the toothbrush according to the invention, during its use, handle 3 sends to the toothbrush 4 a suitable magnetic signal.

The operation is ensured by the built-in battery B, having rechargeable elements.

Once terminated its use, handle 3 must be put on the base 2, and automatically the sending of the signal to the toothbrush 4 will finish, and the charging of the battery B will start.

Lifting again the handle 3 from the base 2, the sending of the signal to the toothbrush will occur again, said sending being confirmed by the flashing of a little pilot lamp (not shown) provided close to the coupling of the toothbrush 4 on the handle 3.

If battery B is not sufficiently charged, its operation is automatically stopped and the pilot lamp does not flash.

It is well evident that the base 2 can be always coupled to the network feeding outlet A.

It is possible to completely switch off the inner circuit approaching a little blocking magnet (not shown) (as equipment) within the suitable handle housing, thus allowing its transportation, storing, and in any case among inactivity periods.

In the following a possible solution for the electronic circuit of the toothbrush according to the invention will be described. However, it must be taken into account that it only is a possible embodiment, the same not being in any way limitative of the scope of the invention.

The electronic circuit is provided within the handle 3, and has, beside the task of generating the signal for the toothbrush 4, also that of checking the charge of the battery B and the selection of the two operations.

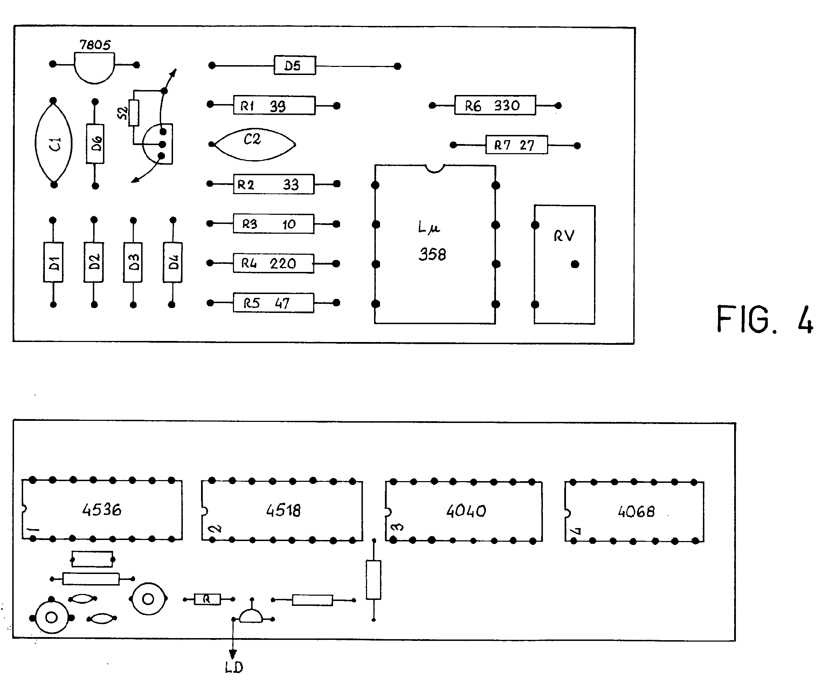

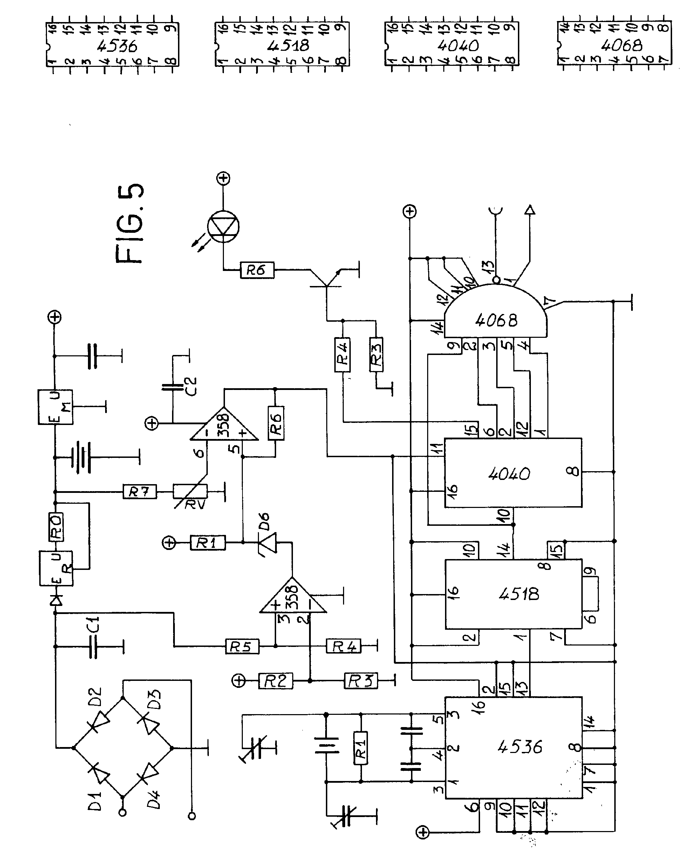

In general terms, and observing figures 4 and 5, the circuit is comprised of four stages, namely:
- a recharging tension detector and inhibitor of the oscillation stage (in the control unit C);
- a recharging current limiting device and a controller of the battery B tension (in the feeding unit);
- a generator dividing the clock signal (in the oscillator);
- a frequency generator and mixer, preferably five frequencies, depending on the clock (in the oscillator).

When the handle 3 is placed on the base 2, the secondary winding within the same receives tension induced by the primary winding provided within the base 2. Said tension is sent to the first one of the two operational amplifiers contained within the integrated circuit of the first stage, a double low consumption operational circuit (e.g. of the TL 082 kind). The latter blocks the third stage comprised of an oscillator - divider integrated circuit (a CD 4536) with the recharging induced tension or with the discharged battery.

Third stage generates a clock divided by ten, one hundred or one thousand, being 100 the preferred value, by a divider integrated circuit (e.g. of the CD 4518 type).

The fourth stage (comprised of a CD 4040) will fraction this frequency into other frequencies, preferably into five frequencies, that will be elaborated by the last stage (a HEF 4068) also acting as exit buffer.

Particularly, in the preferred embodiment of the toothbrush according to the invention, said frequencies are included in the range between 0 and 2500 Hz, and particularly 1972.63 Hz, 246.58 Hz, 30.822 Hz, 3852 Hz, 0.481 Hz. The constant of division is equal to 8.

The present invention has been described for illustrative but not limitative purposes, according to its preferred embodiments, but it is to be understood that modifications and/or changes can be introduced by those skilled in the art without departing from the relevant scope as defined in the enclosed claims.

## Claims

1. Improved toothbrush, characterised in that it comprises a base element, an handle and a replaceable toothbrush, provided with bristles, and an electronic circuit, said circuit comprising a feeding, provided in said base element, a control unit, an oscillator generator and mixer of frequencies depending on the clock, provided within the handle, said toothbrush acting as antenna.

2. Improved toothbrush according to claim 1, characterised in that frequencies included in the range between 0 and 2500 Hz are generated and mixed, preferably five frequencies.

3. Improved toothbrush according to claim 2, characterised in that Still more preferably, said frequencies are 1972.63 Hz, 246.58 Hz, 30.822 Hz, 3852 Hz, 0.481 Hz, with a division constant equal to 8.

4. Improved toothbrush according to one of the preceding claims, characterised in that a rechargeable battery is provided.

5. Improved toothbrush according to one of the preceding claims, characterised in that said control unit provides an enabling and de-enabling element of the toothbrush.

6. Improved toothbrush according to one of the preceding claims, characterised in that an activation pilot lamp is provided in said handle.

7. Improved toothbrush according to one of the preceding claims, characterised in that said circuit provides four stages, namely:
- a recharging tension detector and inhibitor of the oscillation stage;
- a recharging current limiting device and a controller of the battery B tension;
- a generator dividing the clock signal;
- a frequency generator and mixer, preferably five frequencies, depending on the clock.

8. Improved toothbrush according to each one of the preceding claims, substantially as illustrated and described.
